# EUROPEAN PATENT APPLICATION

(11) **EP 4 464 323 A1**
(43) Date of publication of application: **20.11.2024**
(21) Application number: 22920102.5
(22) Date of filing: 30.12.2022
(51) Int. Cl.: A61K 31/704, A61P 31/14, A61P 11/00, A61P 29/00

(54) **ANTI-INFLAMMATORY USE OF GINSENOSIDE RD**

(30) Priority: 11.01.2022 CN 202210027815
(71) Applicant: Fu, Li, Dalian, Liaoning 116100 (CN)
(72) Inventor: SUI, Dayun, Dalian, Liaoning 116100 (CN); XU, Huali, Dalian, Liaoning 116100 (CN); LU, Mingming, Dalian, Liaoning 116100 (CN); YU, Xiaofeng, Dalian, Liaoning 116100 (CN); WANG, Shuo, Dalian, Liaoning 116100 (CN); FU, Wenwen, Dalian, Liaoning 116100 (CN); FENG, Xue, Dalian, Liaoning 116100 (CN); LI, Yuangeng, Dalian, Liaoning 116100 (CN); SUN, Baohua, Dalian, Liaoning 116100 (CN); FU, Wenfei, Dalian, Liaoning 116100 (CN); LIN, Rongxin, Dalian, Liaoning 116100 (CN); LIU, Yang, Dalian, Liaoning 116100 (CN); GE, Xin, Dalian, Liaoning 116100 (CN); FU, Li, Dalian, Liaoning 116100 (CN)
(74) Representative: Murgitroyd & Company
(86) International application number: PCT/CN2022/143825
(87) International publication number: WO 2023/134472

(57) **Abstract**

The present invention discloses a novel application of ginsenoside Rd in preparation of a drug for relieving or/and treating inflammations and health food/medicinal and edible food/functional food/dietary supplement. Tests prove that ginsenoside Rd has a remarkable curative effect on inflammations, takes effect quickly, has a better effect than Dexamethasone, is suitable for both acute inflammations such as influenza virus pneumonia, novel coronavirus pneumonia and acute lung injury inflammations, and chronic inflammations such as chronic obstructive pneumonia and chronic asthmatic airway inflammations, and is regarded as a new drug source for treating acute and chronic inflammations and complications thereof.

## Description

### Technical Field

The present invention relates to the technical field of medicine, relates to an anti-inflammatory drug or health food/medicinal and edible food/functional food/dietary supplement, and more particularly, relates to an application of ginsenoside Rd in preparation of an anti-inflammatory drug or health food/medicinal and edible food/functional food/dietary supplement.

### Background Art

Inflammation is a protective response of the body against external infections or noxious stimulation, and is also a common pathological process of many different types of diseases. Long-term persistent and excessive inflammatory response or inflammatory storm will cause serious damages to the body itself, and even organ failure, so drug intervention is required to block the continuation and development of inflammations. At present, there are two main types of anti-inflammatory drugs commonly used in clinical practice: non-steroidal anti-inflammatory drugs and steroidal anti-inflammatory drugs (glucocorticoids). These two types of anti-inflammatory drugs have good clinical anti-inflammatory effects, but will produce a series of adverse reactions and tolerances after long-term massive use, e.g., the former causes gastric mucosal injuries, liver and kidney damages and cardiac toxic and side effects, and the latter causes water and sodium retention. In order to solve the tolerances and adverse reactions of drugs, the search for anti-inflammatory drugs with novel structures and unique mechanisms has always been a hot topic in the field of research and development of novel anti-inflammatory drugs. Ginsenoside Rd belongs to a diol type of ginsenoside, which is mainly found in Radix Ginseng, Radix Notoginseng, and Radix Panacis Quinquefolii. It has a wide range of biological activities, obvious pharmacological effects on heart and cerebral vessels, nervous systems, immune systems, etc., as well as the effects better than other monomeric saponins in analgesia and neuroprotection, but its anti-inflammatory and protective effects and mechanisms against acute inflammation (ALI) have not been reported in literatures.

### Summary of the Invention

A primary objective of the present invention is to provide anti-inflammatory properties and efficacy of ginsenoside Rd in view of the technical problems existing in the above-mentioned existing anti-inflammatory drugs or health food/medicinal and edible food/functional food/dietary supplement, and to provide new medicinal use of the ginsenoside Rd, that is, a novel application of the ginsenoside Rd in an anti-inflammatory drug or health food/medicinal and edible food/functional food/dietary supplement.

To fulfill said objective, in one aspect, the present invention provides an application of ginsenoside Rd in preparation of an anti-inflammatory drug or health food/medicinal and edible food/functional food/dietary supplement.

In the process of screening natural active ingredients with anti-inflammatory effects, the inventors have found that ginsenoside Rd in the chemical composition of Radix Ginseng has a significant anti-inflammatory effect.

The drug is used for the treatment of one or more of acute inflammations and chronic inflammations.

The drug or health food/medicinal and edible food/functional food/dietary supplement is composed of ginsenoside Rd and a pharmaceutically acceptable carrier.

The content of the ginsenoside Rd is 1% to 99.9%; preferably, 30 to 99.9%; further preferably, 60 to 99.9%; further preferably, 80 to 99.9%; and preferably, 98 to 99.9%.

In particular, a ratio of a weight of the ginsenoside Rd to a total weight of the preparation is (0.01-100): 100, preferably (0.1-100): 100, and further preferably (10-100): 100.

In particular, the pharmaceutically acceptable carrier is often recognized by health professionals for this objective and as an inactive ingredient in pharmaceuticals. The compilation related to pharmaceutically acceptable carriers can be found in handbooks such as "Handbook of Pharmaceutical Excipients" (2nd Edition, edited by A. Wade and P. J. Weller; published by American Pharmaceutical Association, Washington and The Pharmaceutical Press, London, 1994). In particular, the carrier includes an excipient, such as starch or water; a lubricant, such as magnesium stearate; a disintegrant, such as microcrystalline cellulose; filler, such as lactose; a binder, such as pregelatinized starch or dextrin; a sweetener; an antioxidant; an ointment base; a transdermal promoter; a preservative; a flavoring agent; spices, etc.; and
the drug is an oral preparation, a topical preparation or an injection; the oral preparation is tablets, capsules, granules, pills, a solution, a suspension, an emulsion, an oral liquid, drops, or a syrup; the topical preparation is emplastrum, an ointment or a suppository; and the injection is an injection solution, a powder injection or a lyophilized powder injection.

The health food/medicine and edible food/functional food/dietary supplement is an oral preparation; and the oral preparation is tablets, a powder, granules, medicinal tea, pills, a paste, hard capsules, soft capsules, an oral liquid, medicated wine, a syrup, or drops.

In another aspect, the present invention provides an anti-inflammatory drug or health food/medicinal and edible food/functional food/dietary supplement, wherein the drug or health food/medicinal and edible food/functional food/dietary supplement contains ginsenoside Rd.

The content of the ginsenoside Rd is 1% to 99.9%; preferably, 30 to 99.9%; further preferably, 60 to 99.9%; further preferably, 80 to 99.9%; and preferably, 98 to 99.9%.

In particular, a ratio of a weight of the ginsenoside Rd to a total weight of the preparation is (0.01-100): 100, preferably (0.1-100): 100, and further preferably (10-100): 100.

In particular, the drug or health food/medicinal and edible food/functional food/dietary supplement further includes one or more of a Radix Scrophulariae extract, a Rehmannia glutinosa Libosch extract, a Radix Ophiopogonis extract, a Radix Scutellariae extract, a Radix Ranunculi Ternati extract, a Fritillaria extract, a Menthae Haplocalycis Herba extract, a Semen Sesami Nigrum extract, a Zingiber officinale Roscoe extract, a grape seed extract, a pomegranate seed extract, a Rhizoma Cyperi extract, a Pericarpium Citri Reticulatae Viride extract, plant essential oil, arbutin, vitamin C and derivatives thereof, or vitamin E and derivatives thereof.

The drug may be prepared into various dosage forms by methods well-known in the art, such as tablets, capsules, pills, powder, granules, a syrup, emplastrum, an ointment, or a suppository. Compared with the prior art, the present invention has the following obvious advantages.
1. The present invention has discovered a new medicinal value for the known compound ginsenoside Rd, which is used for an anti-inflammatory purpose, and can be prepared into an anti-inflammatory drug or health food/medicinal and edible food/functional food/dietary supplement, thus opening up a new field for the application of Radix Ginseng medicinal materials.
2. A series of experimental studies of the present invention prove that the ginsenoside Rd has a significant anti-inflammatory effect.
3. The ginsenoside Rd of the present invention has a strong anti-inflammatory pharmacological effect, takes effect quickly, has small toxic and side effects and good safety, can be taken for a long time, and has a good medicinal prospect.
4. The product of the present invention is abundant and cheap in raw materials, safe for clinical use, and simple in preparation process, can be prepared into various dosage forms, and is small in dosage, convenient to use, and thus easy to promote.
5. According to the present invention, the single-component ginsenoside Rd (an anti-inflammatory active ingredient) can be used to prepare an anti-inflammatory drug, or a composite drug for the treatment of inflammations can also be prepared by a combined formulation of the ginsenoside Rd and other active ingredients (e.g., a Radix Notoginseng, a Radix et Rhizoma Tripterygii extract, a Radix Bupleuri extract, a powered Radix Stephaniae Tetrandrae extract, a Radix Scutellariae extract, a Radix Sophorae Flavescentis extract, a Melilotus suaveolens Ledeb extract, a grape seed extract, a pomegranate seed extract, vitamin C and its derivatives, or vitamin E and its derivatives).

### Detailed Description of the Invention

The beneficial effects of the formula of the present invention are further described below by specific embodiments, and these examples include pharmacodynamic tests for capsules, tablets and soft capsules of ginsenoside Rd of the present invention. These examples are only exemplary and do not constitute any limitation on the scope of the present invention. Those skilled in the art should understand that the details and forms of the technical solutions of the present invention may be modified or replaced without deviating from the formula idea and the use scope of the present invention, but these modifications and substitutions fall within the protection scope of the present invention.

### Example 1 Ginsenoside Rd tablets

Ginsenoside Rd tablets were prepared in the following proportions:

| | |
|---|---|
| Ginsenoside Rd (98% in content) | 500 g |
| Starch | 1000 g |
| Talcum powder | 1% (15g) |
| Magnesium stearate | 1% (15g) |

Ginsenoside Rd and starch were mixed well and prepared into granules, and the granules were added with talcum powder and magnesium stearate, mixed well and pressed into 10,000 tablets.

### Example 2 Ginsenoside Rd capsules

1. Raw materials were prepared according to the following proportions:

| | |
|---|---|
| Ginsenoside Rd (98% in purity) | 500 g |
| Starch | 1000 g |

Ginsenoside Rd and starch were mixed well and packed into 10,000 capsules.

### Example 3 Ginsenoside Rd granules

1. Raw materials were prepared according to the following proportions:

| | |
|---|---|
| Ginsenoside Rd (63% in purity) | 10 g |
| Starch | 1000 g |

Ginsenoside Rd and starch were mixed well and prepared into granules, which were then loaded into 10,000 bags.

### Example 4 Ginsenoside Rd tablets

1. Raw materials were prepared according to the following proportions:

| | |
|---|---|
| Ginsenoside Rd (63% in purity) | 30 g |
| Rehmannia glutinosa Libosch extract | 20 g |
| Radix Ophiopogonis extract | 40 g |
| Starch | 1000 g |
| Talcum powder | 1% (14.5 g) |
| Magnesium stearate | 1% (14.5 g) |

Ginsenoside Rd, Rehmannia glutinosa Libosch extract, Radix Ophiopogonis extract and starch were mixed well and prepared into granules, and the granules were added with talcum powder and magnesium stearate, mixed well and pressed into 10,000 tablets.

### Example 5 Ginsenoside Rd capsules

1. Raw materials were prepared according to the following proportions:

| | |
|---|---|
| Ginsenoside Rd (63% in purity) | 30 g |
| Fritillaria extract | 30 g |
| Vitamin C | 600 g |
| Starch | 1000 g |

Ginsenoside Rd, Fritillaria extract, Vitamin C and starch were mixed well and packed into 10,000 capsules.

### Example 6 Ginsenoside Rd granules

| | |
|---|---|
| Ginsenoside Rd (63% in purity) | 30 g |
| Radix Scutellariae extract | 30 g |
| Vitamin C | 600 g |
| Powdered sucrose | 5000 g |

Ginsenoside Rd, Radix Scutellariae extract, Vitamin C and powdered sucrose were mixed well and prepared into granules, which were then loaded into 10,000 bags.

### Example 7 Ginsenoside Rd oral liquid

| | |
|---|---|
| Ginsenoside Rd (63% in content) | 4% |
| Rhizoma Cyperi extract | 3% |
| Pericarpium Citri Reticulatae Viride extract | 3% |
| Glucose syrup | 5% |

Deionized water was finally added to the whole formula to 100%.

Ginsenoside Rd, Rhizoma Cyperi extract and Pericarpium Citri Reticulatae Viride extract were dissolved with ethanol, and added with glucose syrup to 100% to obtain the finished product.

### Example 8 Ginsenoside Rd solid drink

| | |
|---|---|
| Ginsenoside Rd (50% in purity) | 30 g |
| Radix Scutellariae extract | 30 g |
| Vitamin C | 600 g |
| Powdered sucrose | 5000 g |

Ginsenoside Rd, Radix Scutellariae extract, Vitamin C and powdered sucrose were mixed well and prepared into granules, which were then loaded into 10,000 bags.

### Example 9 Ginsenoside Rd tableted candies

| | |
|---|---|
| Ginsenoside Rd (10% in purity) | 600 g |
| Xylitol | 350 g |
| Citric acid | 10 g |
| Aspartame | 15 g |
| Magnesium stearate | 10 g |
| Menthae Haplocalycis Herba essence | 15 g |

Ginsenoside Rd, xylitol, citric acid, aspartame and Menthae Haplocalycis Herba essence were mixed well and prepared into granules, and the granules were added with magnesium stearate, mixed well and pressed into 1000 tablets.

### Example 10 Ginsenoside Rd ointment

| | |
|---|---|
| Ginsenoside Rd (63% in content) | 100 g |
| Aqueous phase (methylparaben, water) | 250 g |
| Oil phase (octadecanol, vaseline) | 1000 g |

The aqueous phase was added to the oil phase, stirred, and added with ginsenoside Rd to obtain the finished product.

### Experimental example 1 Effect of ginsenoside Rd on xylene-induced swelling of mouse ears

### 1.1 Experimental animals and pharmaceuticals

### 1.1.1 Experimental animals:

Male and female Kunming mice (SPF grade), weighing 18-22g, were purchased from Liaoning Changsheng Biotechnology Co., Ltd., with quality certificate number: SCXK-(Liaoning) 2020-0001.

### 1.1.2 Reagents and tested drugs:

Dexamethasone, xylene, purchased from Shanghai Chemical Reagent Company; ginsenoside Rd (content ≥ 95%), produced by Dalian Fusheng Natural Medicine Development Co., Ltd., detected by HPLC with Ginsenoside Rd provided by China Institute for Food and Drug Control as a control, and having a content of 96.2%.

### 1.2 Experimental method

Twenty KM mice, weighing 18-22 g, were randomly grouped, with ten mice in each group. The groups include a model group, a positive control group (Dexamethasone, 2 mg/kg), a ginsenoside Rd low-dose group (5 mg/kg), and a ginsenoside Rd high-dose group (10 mg/kg). After 30 min of gavage administration of mice (the model group was administrated with the same volume of 0.5% sodium carboxymethylcellulose by gavage), 0.03 ml of xylene was evenly applied to both sides of the right ear of each mouse in each group to cause inflammations on both sides, and the left ear of the mouse was not applied as a control. After 4 h, the mouse was killed by dislocation of cervical vertebra, the two ears of the mouse were cut off along the auricle, round ear slices of the same size (a diameter of 6 mm) were taken at the same position of the left and right ears and weighed respectively, a mass difference of the two ear slices was used as an ear swelling degree, and a swelling inhibition rate was calculated. Ear swelling rate (%) = [right ear weight (swollen ear) - left ear weight (control ear)] / left ear weight (control ear) × 100%. Swelling inhibition rate (%) = [(ear swelling rate in the model group - ear swelling rate in the test group)/ear swelling rate in the model group] ×100%.

### 1.3 Results and Conclusions

**Table 1-1 The results of ear mass difference, ear swelling rate and ear swelling inhibition rate of mice in various groups**

| **Group** | **Dose** | **Ear mass difference (mg)** | **Ear swelling rate (%)** | **Ear swelling inhibition rate (%)** |
|---|---|---|---|---|
| Model group | -- | 16.37±1.17 | 112.05 | -- |
| Dexamethasone group | 2 mg/kg | 10.87 ±0.53 | 69.68 | 37.81 |
| Ginsenoside Rd low-dose group | 5 mg/kg | 11.84 ±1.02 | 75.56 | 32.57 |
| Ginsenoside Rd high-dose group | 10 mg/kg | 7.68 ±0.39** | 48.64** | 56.59 ** |

| | | | | |
|---|---|---|---|---|
| Compared with a positive drug group, **P<0.01 | | | | |

Compared with the model group, the ginsenoside Rd drug group could significantly inhibit ear swelling of mice caused by xylene, and had a significant anti-inflammatory effect. The ear swelling inhibition rate at a dose of 10 mg/kg was significantly better than that of Dexamethaone, achieving a more obvious anti-inflammatory effect.

### Experimental example 2 Effect of ginsenoside Rd on pneumonia caused by mouse influenza virus

### 1.1 Experimental animals and pharmaceuticals

### 1.1.1 Experimental animals:

ICR mice, weighing 20-30 g, laboratory animal supplier: Hunan SJA Laboratory Animal Co.,Ltd., laboratory animal production license: SCXK (Xiang) 2019-0004, and feed supplier: Jiangsu Medicience Biopharmaceutical Co., Ltd.

### 1.1.2 Virus and drug:

Influenza virus (BSL-3 Laboratory, Guangzhou Customs Technology Center (Laboratory of Highly Pathogenic Microorganisms, State Key Laboratory of Respiratory Diseases). Ginsenoside Rd (content ≥ 95%), produced by Dalian Fusheng Natural Medicine Development Co., Ltd., detected by HPLC with Ginsenoside Rd provided by China Institute for Food and Drug Control as a control, and having a content of 96.2%.

Dexamethasone, purchased from Shanghai Chemical Reagent Company.

### 1.2 Experimental method

### (1) Virus inoculation

Influenza virus nasal vaccination: a lump of absorbent cotton was put into a beaker of 200-300 mL, and then poured with an appropriate amount of ether (till the absorbent cotton was wetted). The beaker filled with the absorbent cotton was inverted, and added with the mice for anesthesia. When the mice were extremely excited and then obviously weak, that is, the anesthesia depth was moderate, the mice were taken out. The ether-anesthetized mice were placed on their backs, with the mouse head facing upward, and then were subjected to intranasal infection. A diluted influenza virus was added to nostrils at 0.03 mL/nostril (15LD₅₀). A blank control was administrated with normal saline, instead of a virus suspension.

### (2) Administration method

Drug groups (10 mg/kg, 5 mg/kg), a model group, and a Dexamethasone control group (2 mg/kg) were administrated by routine gavage on the day before infection, once a day, for five consecutive days; and the positive blank group and the model group were perfused with the same volume of 0.5% sodium carboxymethylcellulose.

### (3) IL-2 and TNF-a detection

On the Day 5 after influenza virus infection, that is, Day 6 after administration, after the mice were fasted for 8 h and weighed, venous blood was collected from the plexus ophthalmicus, and blood from mice in each group was preserved, subjected to serum separation, and stored in a refrigerator for later use. According to the instructions of a test kit, various detection and analysis were carried out, and IL-2 and TNF-a concentrations were calculated based on the standard detection and standard curve plotting.

### (4) Histological and pathological examination of lung lesions

Rat lungs in various experimental groups were taken for fixation, embedding, section staining, microscopic examination, and the like.

### 1.3. Test results

(1) The pathological results of the lungs of the mice showed that compared with the normal group, the lung tissues of the infected model group presented pathological changes such as pulmonary hemorrhage and interstitial pneumonia. The specific types and degrees of lesions of infected animals in various groups were as follows:

**Table 2-1 Pathomorphological observation results of lungs of mice infected with influena virus**

| Experimental group | Pulmonary hemorrhage | | | Interstitial pneumonia | | |
|---|---|---|---|---|---|---|
| | Mild | Moderate | Severe | Mild | Moderate | Severe |
| Virus group | 1 | 8 | 1 | 2 | 6 | 2 |
| Dexamethasone group | 6 | 4 | 0 | 6 | 4 | 0 |
| Ginsenoside Rd low-dose group | 5 | 5 | 0 | 5 | 5 | 0 |
| Ginsenoside Rd high-dose group | 7 | 3 | 0 | 7 | 3 | 0 |

Compared with the blank group, the pathological changes in the lungs of the animals in the virus group were significant; lesion degrees of the drug low-dose group and the drug high-dose group were milder than those of the virus group, and 10 mg/kg and 5 mg/kg drugs had good improvement effects on lung lesions caused by influenza virus; and the high-dose group had an effect better than Dexamethasone.
(2) The detection results of TNF-α and IL-2 showed that different concentrations of drugs intervened in both TNF-α and IL-2. The efficacy of the high-dose group was better than that of Dexamethasone.

**Table 2-2 Expression levels of TNF-α and IL-2 in lung homogenate of influenza virus-infected mice (x̅±s, n=10)**

| | Group | TNF-α | IL-2 |
|---|---|---|---|
| Model group | -- | 1065.85±18.39 | 153.22±40.42 |
| Blank group | -- | 817.53 ±54.95 | 295.79 ±35.37 |
| Dexamethasone group | 2 mg/kg | 862.85 ±28.87 | 285.20 ±49.02 |
| Ginsenoside Rd high-dose group | 10 mg/kg | 824.09 ±17.57** | 293.83 ±29.44** |
| Ginsenoside Rd low-dose group | 5 mg/kg | 889.87 ±28.58 | 263.66 ±10.25 |

| | | | |
|---|---|---|---|
| Compared with a positive drug group, **P<0.01, *P<0.05 | | | |

### Experimental example 3 Effect of ginsenoside Rd on pneumonia caused by Delta variant of novel coronavirus in mice

### 1.1 Experimental animals and pharmaceuticals

### 1.1.1 Experimental animals:

hACE2 mice, aged 6-7 weeks and weighing 20-40 g, laboratory animal supplier: Jiangsu Gempharmatech Co., Ltd., laboratory animal production license: SCXK (Jiangsu) 2018-0008, and feed supplier: Jiangsu Medicience Biopharmaceutical Co., Ltd.

### 1.1.2 Virus and drug:

COVID-19 Delta virus (BSL-3 Laboratory, Guangzhou Customs Technology Center (Laboratory of Highly Pathogenic Microorganisms, State Key Laboratory of Respiratory Diseases).

Ginsenoside Rd (content ≥ 95%), produced by Dalian Fusheng Natural Medicine Development Co., Ltd., detected by HPLC with Ginsenoside Rd provided by China Institute for Food and Drug Control as a control, and having a content of 96.2%.

Dexamethasone, purchased from Shanghai Chemical Reagent Company.

### 1.2 Experimental method

1.2.1 hACE2 transgenic C57BL/6 mice were divided into a normal group, a COVID-19 De1ta virus infection group, drug groups (a 10 mg/kg low-dose group, and a 20 mg/kg high-dose group), and a positive control group (Dexamethasone, 5 mg/kg), with eight mice in each group. Except for the mice in the normal group who were administrated with PBS by nasal drops, the mice in the other groups were infected with COVID-19 Delta virus in 10⁵ PFU by nasal drops. 2 h after infection, the mice in each drug group were administrated with the drug by gavage for five consecutive days, once a day. Dead or dying animals were dissected and lungs were picked after euthanasia; and surviving animals were dissected on the Day 5 after infection, and lungs were picked.

1.2.2 In the infection group, four animals in each group were dissected for lung tissues for pathological study. Four animals were dissected, and lung tissues were picked. Total RNA was extracted from a homogenate supernatant of the lung tissues by a Trizol method, and inflammatory factor interleukin 1β (IL-1β) and monocyte chemoattractant protein 1 (MCP-1) were detected by RT-qPCR.

1.2.3 The specific operation steps of histopathological study of lung tissues were as follows:
(1) fixation and embedding: fixation (fixed with 4% paraformaldehyde) → washing and trimming, trimming tissue to obtain a flat section→ dehydration with 50%, 70%, 80%, 95% and 100% ethanol solutions→ immersion in xylene I and xylene II respectively for transparentizing → immersion with 60°C wax I and II respectively for wax penetration → paraffin embedding; and
(2) slice staining: sectioning (a thickness of about 4 um) → immersion of paraffin section in a clarifying agent I, a clarifying agent II, and a clarifying agent III for deparaffinization → soaking in 100%, 90%, 80%, 70% and other levels of alcohol solutions for 5 min each → washing with water → staining with hematoxylin for 3 min → washing with water → differentiating with 0.5% hydrochloric acid alcohol for a few seconds → washing with tap water for 15 min for bluing → staining with eosin for 2 min → immersion in 95% ethanol I, 95% ethanol II, 100% ethanol I, 100% ethanol II, a clarifying agent I, a clarifying agent II, and a clarifying agent III respectively for dehydration and transparentizing → sealing with neutral gum for storage → observing and photographing under a microscope.

### 1.3. Test results

(1) The pathological results of the lungs of the mice showed that compared with the normal group, the lung tissues of the infected model group presented pathological changes such as pulmonary hemorrhage and interstitial pneumonia. The specific types and degrees of lesions of infected animals in various groups were as follows:

**Table 3-1 Pathomorphological observation results of lungs of mice infected with COVID-19 Delta virus**

| Group | Pulmonary hemorrhage | | | Interstitial pneumonia | | |
|---|---|---|---|---|---|---|
| | Mild | Moderate | Severe | Mild | Moderate | Severe |
| Virus group | 0 | 4 | 0 | 0 | 3 | 1 |
| Dexamethasone group | 3 | 1 | 0 | 3 | 1 | 0 |
| Ginsenoside Rd low-dose group | 2 | 2 | 0 | 2 | 2 | 0 |
| Ginsenoside Rd high-dose group | 4 | 0 | 0 | 4 | 0 | 0 |

Based on the comparison of the above lesions in various groups, the pathological changes of the surviving animals in various groups were milder than those of the dead animals; lesion degrees of the drug low-dose group and the drug high-dose group were milder than those of a virus group, and 10 mg/kg and 20 mg/kg drugs had good improvement effects on lung lesions caused by COVID-19 Delta virus; and the drug administrated to the high-dose group was better than Dexamethasone in the pathological improvement of pulmonary hemorrhage and interstitial pneumonia.
(2) The mRNA expression results of lung inflammatory factors showed that on the Day 5 of COVID-19 De1ta virus infection for mice, the expressions of IL-1β and MCP-1 in a virus model group were significantly higher than that in the normal group; compared with the virus model group, IL-1β and MCP-1 inflammatory factors in the ginsenoside Rd drug group were significantly reduced; and the ability to inhibit the overexpression of inflammatory mediators in a 20 mg/kg administration group was superior to that of Dexamethasone.

**Table 3-2 Expression levels of IL-1β and MCP-1 in lung homogenates of COVID-19 De1ta virus-infected mice (x̅±s)**

| Group | Dose | IL-1β | MCP-1 |
|---|---|---|---|
| Blank group | -- | 1.00±0.000 | 1.00±0.000 |
| Model group | -- | 46.52±9.34 | 14.98±11.35 |
| Dexamethasone group | 5 mg/kg | 9.26±10.27 | 6.27±8.20 |
| Ginsenoside Rd high-dose group | 20 mg/kg | 7.13±12.68 | 4.95±14.36 |
| Ginsenoside Rd low-dose group | 10 mg/kg | 9.88±6.42 | 6.71±13.75 |

### Experimental example 4 Effect of ginsenoside Rd on inflammatory response in mice with acute lung injury

### 1.1 Experimental animals and pharmaceuticals

### 1.1.1 Experimental animals:

Balb/c mice, male, weighing 20-22 g, were purchased from Liaoning Changsheng Biotechnology Co., Ltd., with quality certificate number: SCXK-(Liaoning) 2020-0001.

### 1.1.2 Reagents and tested drugs:

propylene glycol, purchased from Shanghai Macklin Biochemical Technology Co., Ltd.;
normal saline, purchased from Kunming Nanjiang Pharmaceutical Co., Ltd.;
lipopolysaccharide, Dexamethasone purchased from Sigma Corp. of America;
pentobarbital sodium, purchased from Shanghai Chemical Reagent Company;
enzyme-linked immunosorbent assay kit, mouse TNF-α detection kit, mouse IL-1β detection kit, mouse IL-6 detection kit, and mouse IL-18 detection kit, purchased from NanJing JianCheng Bioengineering Institute; and
test drug: ginsenoside Rd (content ≥ 95%), produced by Dalian Fusheng Natural Medicine Development Co., Ltd., detected by HPLC with Ginsenoside Rd provided by China Institute for Food and Drug Control as a control, and having a content of 96.2%.

### 1.2 Experimental method

### 1.2.1 Experimental grouping and administration

Balb/c male mice, weighing 20-22 g, were randomly divided into five groups according to body weights, with eight mice in each group: a blank control group (propylene glycol-0.5% carboxymethylcellulose sodium), a model group (LPS, 15 mg/kg), a ginsenoside Rd low-dose group (10 mg/kg), a ginsenoside Rd high-dose group (20 mg/kg), and a Dexamethasone group (5 mg/kg). Mice in each group were intraperitoneally injected; after 1 h of administration, except for the blank control group, the mice in each group were intratracheally instilled with 20 µg of LPS for modeling; and the blank control group was intratracheally instilled with an equal volume of PBS.

### 1.2.2 Establishment of mouse ALI model

After 1 h of administration, a mouse ALI model was established; each mouse was intraperitoneally injected with sodium pentobarbital at 30 mg/kg, anesthetized and fixed on an operating table dorsally; skin preparation was carried out on the neck; the skin was incised to expose the trachea; 20 µg of LPS was instilled into the trachea using a microglass syringe; and then the mouse was gently rotated head up on the operating table, such that the instilled liquid was evenly distributed in the mouse lungs, and the skin incision was sutured.

### 1.2.3 Determination of TNF-α, IL-1β, IL-6 and IL-18 content in lung tissues and BLAF

After 24 h of modeling, anesthetized mice were intraperitoneally injected with sodium pentobarbital at 30 mg/kg; lung tissues of the mice were taken and rinsed repeatedly in pre-cooled normal saline; the lung tissues were blood-removed, wiped with filter paper, weighed, and added with normal saline according to a ratio of 1:9 (W:V=1 g:9 ml); a tissue homogenate was prepared with a homogenization machine under an ice water bath; and a supernatant of mouse lung tissue homogenate was taken.

The mouse was anesthetized, and the trachea was separated; the whole lung was lavaged with 0.5 ml of pre-cooled normal saline, stayed for 1 min, and slowly pumped back with a syringe, and this process was repeated three times; and BLAF was collected, and centrifuged for 15 min at 4°C and 1600 rpm/min.

In strict accordance with the operating instructions of an enzyme-linked immunosorbent assay kit (ELISA), the contents of TNF-α, IL-1β, IL-6 and IL-18 in the supernatant of mouse lung tissue homogenate and the BLAF supernatant were detected.

### 1.3 Results

Compared with the blank control group, the contents of TNF-α, IL-1β, IL-6 and IL-18 in lung tissues and alveolar lavage fluid of each mouse in the model group were significantly increased (P<0.01); and compared with the model group, the ginsenoside Rd drug group and the Dexamethasone group could significantly reduce the contents of TNF-α, IL-1β, IL-6 and IL-18 in the lung tissues and BLAF of the mice, and the effect of the ginsenoside Rd high-dose group was better than that of Dexamethasone (P<0.01).

**Table 4-1 Effects of ginsenoside Rd on the contents of inflammatory factors in lung tissue of mice with acute lung injury (X±SD, n=8)**

| Group | Dose | TNF-α (ng/L) | IL-1β (ng/L) | IL-6 (ng/L) | IL-18 (ng/L) |
|---|---|---|---|---|---|
| Blank group | -- | 39.67±12.05 | 31.03±8.62 | 28.62±2.03 | 28.00±6.35 |
| Model group | -- | 115.26±20.25 | 103.33±23.26 | 101.96±14.89 | 117.28±21.22 |
| Ginsenoside Rd low-dose group | 10 mg/kg | 98.14±14.48 | 80.23±18.08 | 84.70±10.84 | 94.03±15.22 |
| Ginsenoside Rd high-dose group | 20 mg/kg | 46.88±19.52** | 30.52±13.42** | 36.81±13.45** | 37.54±15.80* |
| Dexamethaso ne group | 5 mg/kg | 58.64±10.65 | 48.76±12.76 | 49.73±16.08 | 49.06±15.41 |

| | | | | | |
|---|---|---|---|---|---|
| Compared with a positive drug group, **P<0.01, *P<0.05 | | | | | |

**Table 4-2 Effects of ginsenoside Rd on the contents of inflammatory factors in alveolar lavage fluid of mice with acute lung injury (X±SD, n=8)**

| Group | Dose | TNF-α (ng/L) | IL-1β (ng/L) | IL-6 (ng/L) | IL-18 (ng/L) |
|---|---|---|---|---|---|
| Blank group | -- | 159.64±46.99 | 42.74±13.67 | 21.34±8.17 | 35.43±8.35 |
| Model group | -- | 475.22±82.77 | 105.03±25.52 | 55.03±13.82 | 108.09±34.55 |
| Ginsenoside Rd low-dose group | 10 mg/kg | 344.09±61.08 | 79.63±21.33 | 39.37±9.12 | 81.20±21.27 |
| Ginsenoside Rd high-dose group | 20 mg/kg | 186.04±75.00** | 49.51±6.89** | 29.53±8.99* | 44.64±17.91** |
| Dexamethasone group | 5 mg/kg | 222.58±60.91 | 58.56±16.24 | 36.89±6.16 | 56.38±9.40 |

| | | | | | |
|---|---|---|---|---|---|
| Compared with a positive drug group, **P<0.01, *P<0.05 | | | | | |

### Experimental example 5 Effect of ginsenoside Rd on chronic obstructive pulmonary disease in rats

### 1.1 Experimental animals and pharmaceuticals

### 1.1.1 Experimental animals:

SD rats, male, weighing 200±20 g, laboratory animal supplier: Hunan SJA Laboratory Animal Co.,Ltd., laboratory animal production license: SCXK (Xiang) 2019-0004, and feed supplier: Jiangsu Medicience Biopharmaceutical Co., Ltd.

### 1.1.2 Drug:

Ginsenoside Rd (content ≥ 95%), produced by Dalian Fusheng Natural Medicine Development Co., Ltd., detected by HPLC with Ginsenoside Rd provided by China Institute for Food and Drug Control as a control, and having a content of 96.2%.

Dexamethasone, purchased from Shanghai Chemical Reagent Company.

Lipopolysaccharide (LPS, lot number: 08168), purchased from Sigma Corp.

TNF-α enzyme-linked immunosorbent assay kit, Nanjing Jiancheng Biotechnology Co., Ltd.
IL-6 ELISA assay kit, Nanjing Jiancheng Biotechnology Co., Ltd

### 1.2 Experimental method

### (1) Animal grouping

After adaptive feeding (20-25°C, a humidity of 50-60%) for 1 week, the rats were randomly divided into a blank group, a model group, a positive control group, a drug high-dose group, and a drug low-dose group, with twelve rats in the model group and ten rats in other groups each.

### (2) Model preparation

Except for the blank group, the other groups were treated with fumigation combined with intratracheal injection of lipopolysaccharide to prepare a chronic obstructive pulmonary disease model.

Smoking method: the rat was placed in an organic glass box and smoked for 1 h a day, with 25 cigarettes each time for four consecutive weeks.

Lipopolysaccharide injection method: on the Day 1, Day 8, Day 15 and Day 22 of the smoking day, each rat was anesthetized with 2% sodium pentobarbital (30 mg/kg); the head was fixed in a low position; the glottis was exposed; 0.1 ml of (1mg/ml) lipopolysaccharide was quickly injected into the trachea; and the rat was kept upright, and swung left and right for, such that the lipopolysaccharide was evenly distributed in the two lungs.

Model validation: at the end of smoking, two rats in the model group were taken and killed, and their lung tissues were dissected to observe pathological changes. The lung tissues of the rats showed obvious pathological states, the lung epithelial cells were degenerated and necrotic, and the intercellular space was accompanied by a large number of inflammatory cell infiltration, indicating that the modeling was successful.

### (3) Administration method

The drug high-dose group (10 mg/kg), the low-dose drug group (5 mg/kg), and the Dexamethasone control group (2 mg/kg) were administrated with a drug prepared from 0.5% sodium carboxymethyl cellulose, at a volume of gavage of 10 ml/kg, once a day for twelve consecutive days. The model group and the blank group were administrated with an equal volume of 0.5% sodium carboxymethylcellulose.

### (4) TNF-α and IL-6 detection

After the last administration, the rats were intraperitoneally injected with 2% sodium pentobarbital, anesthetized, and fixed in supine position; 5 ml of blood was collected from the abdominal aorta, and the serum was taken; in strict accordance with the actual instructions, the levels of TNF-a and IL-6 in the serum were detected by enzyme-linked immunosorbent assay. The data was expressed as x̅±s, p<0.05, which meant that it was statistically significant.

### (5) Histopathological examination of lung tissues

Rat lungs in various experimental groups were taken for fixation, embedding, section staining, microscopic examination, and the like.

### 1.3. Test results

(1) TNF-a and IL-6 detection
(2) The detection results of TNF-a and IL-6 showed that different concentrations of drugs intervened in both TNF-a and IL-6. The efficacy of the high-dose group was better than that of Dexamethasone.

**Table 5-1 Expression levels of TNF-a and IL-6 in serum of rats (x̅±s, n=10)**

| | Group | TNF-α | IL-6 |
|---|---|---|---|
| Blank group | -- | 76.04 ±15.84 | 152.93±12.73 |
| Model group | -- | 239.78 ±15.79 | 483.41 ±8.92 |
| Dexamethasone group | 2 mg/kg | 93.71 ±13.27 | 189.65±11.56 |
| Ginsenoside Rd high-dose group | 10 mg/kg | 85.13 ±17.06** | 167.88 ±14.67** |
| Ginsenoside Rd low-dose group | 5 mg/kg | 95.92 ±10.20 | 193.97±13.70 |

| | | | |
|---|---|---|---|
| Compared with a positive drug group, **P<0.01 | | | |

(2) The pathological results of the lungs of the rats showed that compared with the blank group, the lung tissues of the animals in the model group had obvious pathological changes.

**Table 5-2 Pathomorphological observation results of lungs of rats in experimental group**

| Experimental group | Epithelial cell necrosis | | | Interstitial inflammatory cell infiltration | | |
|---|---|---|---|---|---|---|
| | Mild | Moderate | Severe | Mild | Moderate | Severe |
| Model group | 0 | 7 | 3 | 0 | 6 | 4 |
| Dexamethasone group | 7 | 3 | 0 | 6 | 4 | 0 |
| Drug low-dose group | 6 | 4 | 0 | 6 | 4 | 0 |
| Drug high-dose group | 8 | 2 | 0 | 8 | 2 | 0 |

Based on the comparison of the above lesions in various groups, the lesion degrees of the drug low-dose group and the drug high-dose group were milder than that of the model group; 10 mg/kg and 5 mg/kg drugs had good improvement effects on lung lesions; and the high-dose group had an effect superior to Dexamethasone.

### Experimental example 6 Inhibitory effect of ginsenoside Rd on airway inflammations in mice with chronic asthma

### 1.1 Experimental animals and pharmaceuticals

### 1.1.1 Experimental animals:

BALB/c mice, female, weighing 22±2 g, laboratory animal supplier: Hunan SJA Laboratory Animal Co.,Ltd., laboratory animal production license: SCXK (Xiang) 2019-0004, and feed supplier: Jiangsu Medicience Biopharmaceutical Co., Ltd.

### 1.1.2 Drug:

Ginsenoside Rd (content ≥ 95%), produced by Dalian Fusheng Natural Medicine Development Co., Ltd., detected by HPLC with Ginsenoside Rd provided by China Institute for Food and Drug Control as a control, and having a content of 96.2%.

Dexamethasone, purchased from Shanghai Chemical Reagent Company.

Ovalbumin (OVA), purchased from Sigma, Corp.

TNF-α enzyme-linked immunosorbent assay kit, Nanjing Jiancheng Biotechnology Co., Ltd.

IL-6 ELISA assay kit, Nanjing Jiancheng Biotechnology Co., Ltd.

### 1.2 Experimental method

### (1) Animal grouping

After adaptive feeding (20-25°C, a humidity of 50-60%) for 1 week, the mice were randomly divided into a blank group, a model group, a positive control group, a drug high-dose group, and a drug low-dose group, with twelve mice in the model group and ten rats in other groups each.

### (2) Model preparation

Except for the blank group, the mice in the other groups were intraperitoneally injected with 0.2 ml of sensitization solution (containing 3 mg of OVA and 1 mg of aluminum hydroxide) on the Day 1, Day 7 and Day 14, respectively. Starting on Day 21, the mice in each group were placed in a nebulizer and inhaled with 2.5% OVA for excitation. This inhalation was performed once a day, 30 min each time, for ten consecutive days.

On the Day 5 and Day 10 of atomization excitation, one mouse in the model group was taken for model validation.

### (3) Administration method

The drug high-dose group (10 mg/kg), the low-dose drug group (5 mg/kg), and the Dexamethasone control group (2 mg/kg) were administrated with a drug prepared from 0.5% sodium carboxymethyl cellulose, once a day for seven consecutive days.

The model group and the blank group were administrated with an equal volume of 0.5% sodium carboxymethylcellulose.

### (4) Detection:

24 h after the last administration, the mice were killed by inhaling an overdose of ether. The bronchoalveolar lavage fluid (BALF) of the mice was taken and centrifuged, and the supernatant was used for the detection of IL-4, IL-5 and IL-10.

0.5 ml of phosphate buffer was added to the precipitation, resuspended and mixed well; an appropriate amount of suspension was taken and continued to be diluted 20 times with a phosphoric acid sustained-release solution, and a total number of cells was counted under a light microscope; Giemsa staining was performed; and the percentages of neutrophils, eosinophils and lymphocytes were counted.

### 1.3. Test results

### (1) IL-4, IL-5 and IL-10 detection

The results showed that different doses of drugs had intervened in mice with asthma. The efficacy of the high-dose group was better than that of Dexamethasone.

**Table 6-1 Expression levels of IL-4, IL-5 and IL-10 in BALF mice (x̅±s, n=10)**

| | Group | IL-4 | IL-5 | IL-10 |
|---|---|---|---|---|
| Blank group | -- | 7.30 ±11.84 | 11.93±6.74 | 194.30±12.83 |
| Model group | -- | 99.07±12.73 | 36.99 ±12.44 | 81.45±14.32 |
| Dexamethasone group | 2 mg/kg | 9.40 ±13.72 | 15.42 ±6.29 | 172.69±8.53 |
| Ginsenoside Rd high-dose group | 10 mg/kg | 8.11 ±10.98** | 13.14 ±14.75* | 184.48±7.81** |
| Ginsenoside Rd low-dose group | 5 mg/kg | 10.72 ±16.74 | 18.01 ±10.24 | 155.34±13.59 |

| | | | | |
|---|---|---|---|---|
| Compared with a positive drug group, **P<0.01, *P<0.05 | | | | |

### (2) Inflammatory cell count

**Table 6-2 Inflammatory cell count in each group (x̅±s, 10⁵/ml, n=10)**

| | Group | Total cells | Eosinophils | Lymphocyte | Neutrophils |
|---|---|---|---|---|---|
| Blank group | -- | 3.52 ±0.684 | 0.63±0.588 | 0.34±0.972 | 0.41±0.178 |
| Model group | -- | 25.77±1.325 | 6.91±0.542 | 2.03±0.135 | 1.84±0.355 |
| Dexamethasone group | 2 mg/kg | 12.46±0.961 | 1.79±0.273 | 0.90±0.767 | 0.99±0.239 |
| Ginsenoside Rd high-dose group | 10 mg/kg | 9.65±0.231** | 1.41±0.569** | 0.52±0.920* | 0.58±0.255** |
| Ginsenoside Rd low-dose group | 5 mg/kg | 13.89±0.686 | 1.95±0.466 | 0.98±0.848 | 1.16±0.564 |

| | | | | | |
|---|---|---|---|---|---|
| Compared with a positive drug group, **P<0.01, *P<0.05 | | | | | |

The results showed that compared with the blank group, the number of total cells and the number of inflammatory cells in the model group were significantly increased. Different doses of the drug intervened in mice with asthma, and the number of inflammatory cells in the administration group could be significantly reduced compared with the model group. The number of inflammatory cells in the high-dose group was significantly lower than that in the Dexamethasone control group.

## Claims

1. An application of ginsenoside Rd in preparation of a drug for relieving or/and treating acute or chronic inflammations or health food/medicinal and edible food/functional food/dietary supplement.

2. The application according to claim 1, wherein the acute inflammations include influenza virus pneumonia, novel coronavirus pneumonia, acute lung injury inflammations, and the like.

3. The application according to claim 2, wherein a coronavirus is a COVID-19 Delta virus.

4. The application according to claim 1, wherein the chronic inflammations include chronic obstructive pneumonia and chronic asthma airway inflammations.

5. The application according to claim 1, wherein the ginsenoside Rd is cited separately.

6. The application according to claim 1, wherein the relief or treatment effect is better than that of Dexamethasone.

7. The application according to claim 1, wherein the content of the ginsenoside Rd in the drug or health food/functional food/dietary supplement is 1% to 99.9%.

8. The application according to claim 1, wherein the ginsenoside Rd can be extracted and separated from roots, stems, leaves, flowers and fruits of Radix Notoginseng, Radix Ginseng, Radix Panacis Quinquefolii or other plants, and can also be prepared by using processes such as chemical synthesis, chemical transformation and biological transformation.

9. The application according to claim 1, wherein the drug or health food/functional food/dietary supplement is composed of the ginsenoside Rd and a pharmaceutically acceptable carrier or a food-acceptable carrier.

10. The application according to claim 1, wherein the drug or health food/medicinal and edible food/functional food/dietary supplement further contains one or more of a disintegrant, a humectant, a binder, filler, an absorption promoter, a solvent, a lubricant, a surfactant, a flavoring agent, a sweetener, an antioxidant, a preservative and pigment, an ointment base, a transdermal promoter, etc.

11. The application according to claim 1, wherein the drug or health food/medicinal and edible food/functional food/dietary supplement further comprises one or more of a Radix Scrophulariae extract, a Rehmannia glutinosa Libosch extract, a Radix Ophiopogonis extract, a Radix Scutellariae extract, a Radix Ranunculi Ternati extract, a Fritillaria extract, a Menthae Haplocalycis Herba extract, a Semen Sesami Nigrum extract, a Zingiber officinale Roscoe extract, a grape seed extract, a pomegranate seed extract, a Rhizoma Cyperi extract, a Pericarpium Citri Reticulatae Viride extract, plant essential oil, arbutin, vitamin C and derivatives thereof, or vitamin E and derivatives thereof.

12. The application according to any one of claims 9 to 11, wherein a ratio of a weight of the ginsenoside Rd to a total weight of the drug or health food/medicinal and edible food/functional food/dietary supplement is (0.01-100): 100.

13. The application according to claim 1, wherein the drug is an oral preparation, a topical preparation or an injection; preferably, the oral preparation is tablets, capsules, granules, pills, a solution, a suspension, an emulsion, an oral liquid, drops, or a syrup; preferably, the topical preparation is emplastrum, an ointment or a suppository; and preferably, the injection is an injection solution, a powder injection or a lyophilized powder injection.

14. The application according to claim 1, wherein the health food/medicine and edible food/functional food/dietary supplement is an oral preparation; and preferably, the oral preparation is tablets, a powder, granules, medicinal tea, pills, a paste, hard capsules, soft capsules, an oral liquid, medicated wine, a syrup, or drops.
